# EUROPEAN PATENT APPLICATION

(11) **EP 4 661 017 A1**
(43) Date of publication of application: **10.12.2025**
(21) Application number: 23920163.5
(22) Date of filing: 26.12.2023
(51) Int. Cl.: G16H 20/10, G16H 10/20, G16H 50/20, G16H 10/60

(54) **MENTAL ILLNESS DIAGNOSIS AND DRUG RECOMMENDATION METHOD**

(30) Priority: 30.01.2023 KR 20230011636
(71) Applicant: Samsung Life Public Welfare Foundation, Seoul 04348 (KR)
(72) Inventor: LEE, Eun Jin, Seoul 06351 (KR); LEE, Dong Bin, Seoul 06351 (KR); PARK, Woong Yang, Seoul 06351 (KR)
(74) Representative: Weickmann & Weickmann PartmbB
(86) International application number: PCT/KR2023/021521
(87) International publication number: WO 2024/162614

(57) **Abstract**

The present invention relates to a method for mental illness diagnosis and drug recommendation using a mental health questionnaire. The method may provide highly reliable and accurate mental illness diagnosis information and information on medication based on a large amount of data, thereby improving medical quality and reducing medical costs.

## Description

### Technical Field

The present invention relates to a method for mental illness diagnosis and drug recommendation using a mental health questionnaire.

### Background Art

The current estimated global cost of mental health care is US $2.5 trillion per year, and mental health care costs are expected to rise to US $6 trillion by 2030. Diverse care system models have been implemented over the last five decades; however, the issues of cost-effectiveness of, and accessibility to, mental health care remain unresolved. The costs of mental health care include the costs of continuous and updated training for primary care physicians, but a recent survey has found that patient's needs for clinical decision-making still remain unmet.

Artificial intelligence (Al)-based clinical decision support systems (CDSS) might present another potential solution to address these issues. While CDSS have been widely developed in other medical fields and improved practitioners' performance, only a few CDSS have been provided in mental health care thus far. Furthermore, these CDSS have only focused on medication choice based on respective adverse effect profiles, leaving the diagnosis of mental disorders unmet. The current lack of CDSS in mental health care may have originated from the complex and ambiguous processes involved in psychiatric disorders' diagnostic classification, as represented by the Diagnostic and Statistical Manual of Mental Disorders - 5^{th} Edition (DSM-5), as well as to therapeutic decision-making being heavily dependent on individual clinicians' intuition.

Therefore, there are problems in that the current diagnosis of mental illness is dependent on the subjective judgment of clinicians and it is difficult to present clear diagnostic criteria. However, if patients can be classified using data about symptoms and experiences, there is a possibility that mental illness can be diagnosed more objectively. Therefore, there is an urgent need for the development of such an artificial intelligence-based clinical decision support system.

### DISCLOSURE

### Technical Problem

An object of one aspect of the present invention is to provide a method for providing mental illness-related information and drug prescription-related information, including steps of: A) constructing a system for providing mental illness-related information and drug prescription-related information, wherein the system includes: a) a network information providing unit in which a network derivable from mental illness-related questionnaire information is constructed; b) a clustering unit configured to classify mental illness groups based on mental illness-related questionnaire-based information and algorithm information based on the network information derived in a); and c) a drug prescription information deriving unit configured to derive network-based drug prescription information based on the network information derived in a) and cluster-based drug prescription information based on mental illness group information classified in b); and B) inputting patient's questionnaire response information into the system and providing both mental illness-related information based on the mental illness groups classified in the clustering unit b) and drug prescription-related information derived in the deriving unit c).

An object of another aspect of the present invention is to provide a system for providing mental illness-related information and drug prescription-related information, including: a network information providing unit in which a network derivable from mental illness-related questionnaire information is constructed; a clustering unit configured to classify mental illness groups based on mental illness-related questionnaire-based information and algorithm information based on the network information derived in the information providing unit; a cluster-based drug prescription information deriving unit configured to derive cluster-based drug prescription information based on mental illness group information classified in the clustering unit; a network-based drug prescription information deriving unit configured to derive network-based drug prescription information based on network information derived in the information providing unit; and an output unit configured to provide mental illness group information derived in the clustering unit and drug prescription information derived in the network-based drug prescription information deriving unit and the cluster-based drug prescription information deriving unit.

An object of still another aspect of the present invention is to provide a computer-readable recording medium having recorded thereon a program for executing the method or the system.

### Technical Solution

One aspect of the present invention provides a method for providing mental illness-related information and drug prescription-related information, including steps of: A) constructing a system for providing mental illness-related information and drug prescription-related information, wherein the system includes: a) a network information providing unit in which a network derivable from mental illness-related questionnaire information is constructed; b) a clustering unit configured to classify mental illness groups based on mental illness-related questionnaire-based information and algorithm information based on the network information derived in a); and c) a drug prescription information deriving unit configured to derive network-based drug prescription information based on the network information derived in a) and cluster-based drug prescription information based on mental illness group information classified in b); and B) inputting patient's questionnaire response information into the system and providing both mental illness-related information based on the mental illness groups classified in the clustering unit b) and drug prescription-related information derived in the deriving unit c).

In one embodiment, the clustering unit b) may classify mental illness groups based on k-means and Louvain algorithms.

In one embodiment, clustering based on the k-means algorithm may be classification into 9 groups, and clustering based on the Louvain algorithm may be classification into 10 groups.

Another aspect of the present invention provides a system for providing mental illness-related information and drug prescription-related information, including: a network information providing unit in which a network derivable from mental illness-related questionnaire information is constructed; a clustering unit configured to classify mental illness groups based on mental illness-related questionnaire-based information and algorithm information based on the network information derived in the information providing unit; a cluster-based drug prescription information deriving unit configured to derive cluster-based drug prescription information based on mental illness group information classified in the clustering unit; a network-based drug prescription information deriving unit configured to derive network-based drug prescription information based on network information derived in the information providing unit; and an output unit configured to provide mental illness group information derived in the clustering unit and drug prescription information derived in the network-based drug prescription information deriving unit and the cluster-based drug prescription information deriving unit.

In one embodiment, the clustering unit b) in the system for providing mental illness-related information and drug prescription-related information may classify mental illness groups based on k-means and Louvain algorithms.

Still another aspect of the present invention may provide a computer-readable recording medium having recorded thereon a program for executing the method or the system.

### Advantageous Effects

The method for mental illness diagnosis and medication recommendation using a mental health questionnaire according to the present invention may provide highly reliable and accurate mental illness diagnosis information and information on medication based on a large amount of data, thereby improving medical quality and reducing medical costs.

### Brief Description of Drawings

FIG. 1 is a general flow chart showing a method for mental illness diagnosis and drug recommendation.
FIG. 2 shows data showing the comparison of patient numbers between self-reported diagnosis (Dx) and symptom-based diagnosis (Dx) for each mental disorder.
FIG. 3 shows data comparing the distribution of disease combinations between self-reported diagnosis and symptom-based diagnosis.
FIG. 4 shows information regarding diagnosis and results from symptom profiling based on data from 154,348 subjects using UMAP. Specifically, FIG. 4A shows self-reported diagnosis, FIG. 4B shows symptom-based diagnosis, FIG. 4C shows the result of K-mean clustering, and FIG. 4D shows the result of community detection using the Louvain algorithm.
FIG. 5A shows a comparison between k-means (KM) clustering results and Dx (diagnosis) information, FIG. 5B shows a comparison between Louvain (LV) community detection results and Dx information, and FIG. 5C shows a comparison between k-means (KM) clustering results and Louvain (LV) community detection results.
FIG. 6 shows key questions identified in each cluster based on logistic regression.
FIG. 7 shows the distribution of scores for the question categories in each cluster. Specifically, A shows k-means (KM) analysis, and B shows Louvain (LV) analysis.
FIG. 8 shows data showing the frequency of the most frequently prescribed drugs in each drug class.
FIG. 9 shows statistics for cluster-based drug recommendation. Specifically, FIG. 9A shows the percentile of samples with a history of psychiatric drugs for each cluster, and the red line in FIG. 9 shows the overall prescription rate across all clusters (9.12%). FIG. 9B shows data showing the probability of prescribing each drug class to patients in a cluster.
FIG. 10 shows an overview of the network-based drug recommendation system.
FIG. 11 shows drug prescription information of 14,358 individuals in UKB cohort. Specifically, FIG. 11A shows the distribution of the number of prescriptions per person, and FIG 11B shows data showing the proportion of people prescribed with each drug class.
FIG. 12 shows the results of network-based drug recommendation including cluster information. The four bluish bars represent the proportion of people who received recommendations for each drug class in each cluster using several thresholds, i.e., top 1, top 3, top 5, and top 10, and the pink bar represents the percentage of patients who were actually prescribed each drug class. In addition, the left represents KM clustering, and the right represents LV clustering.
FIG. 13 shows data showing the distribution of disease combinations and comparisons between primary diagnosis (Dx) and scale-based diagnosis (Dx) in SMC cohort. Specifically, FIG. 13 shows data showing comparisons between primary diagnosis (Dx) and scale-based diagnosis (Dx) using Sankey plot.
FIG. 14 shows information on drug prescription in SMC cohort. Specifically, FIG. 14A shows the prescription rate of the most frequently prescribed drugs in each drug class, FIG. 14B shows data showing the distribution of the number of drug prescriptions per person, and FIG. 14C shows data showing the proportion of people prescribed with drugs from each drug class.
FIG. 15 shows analysis of SMC cohort. Specifically, FIG. 15 shows information on diagnosis and results from symptom profiling of 842 patients using UMAP. The data show, from left to right, primary diagnosis results, scale-based diagnosis results, the results of k-means analysis, and the results of community detection using the Louvain algorithm.
FIG. 16 shows data showing a comparison across clustering results by primary diagnosis and scale-based diagnosis.
FIG. 17 shows key questions in each cluster as a result of logistic regression. Abbreviations are as follows. CRS: Clinical Rating Scale, HRS: Hamilton Rating Scale, SR: Self-report, APPQ: Albany Panic and Phobia Questionnaire, ASI: Anxiety Sensitivity Index-3, BAI: Beck Anxiety Inventory, BDI: Beck Depression Inventory-II, M.I.N.I: Structured Interview, MDE: Major Depressive Episode.
FIG. 18 shows analysis of SMC cohort. Specifically, it shows data showing the distribution of scores for the question categories in each cluster.
FIG. 19 shows statistics for cluster-based drug recommendation in SMC cohort. Specifically, it shows data showing the percentile of samples with a history of psychiatric drugs for each cluster identified by KM method (A) and LV method (B).
FIG. 20 shows analysis of SMC cohort. Specifically, it shows data showing the probability of prescribing each drug class to patients in a cluster.
FIG. 21 shows analysis of SMC cohort. Specifically, it shows the results of network-based drug recommendation including cluster information. The four bluish bars represent the proportion of people who received recommendations for each drug class in each cluster using several thresholds, i.e., top 1, top 3, top 5, and top 10. The pink bar represents the percentage of patients who were actually prescribed with each drug class. In addition, the left shows data showing KM clustering, and the right shows data showing LV clustering.

### Best Mode

One aspect of the present invention provides a method for providing mental illness-related information and drug prescription-related information, including steps of: A) constructing a system for providing mental illness-related information and drug prescription-related information, wherein the system includes: a) a network information providing unit in which a network derivable from mental illness-related questionnaire information is constructed; b) a clustering unit configured to classify mental illness groups based on mental illness-related questionnaire-based information and algorithm information based on the network information derived in a); and c) a drug prescription information deriving unit configured to derive network-based drug prescription information based on the network information derived in a) and cluster-based drug prescription information based on mental illness group information classified in b); and B) inputting patient's questionnaire response information into the system and providing both mental illness-related information based on the mental illness groups classified in the clustering unit b) and drug prescription-related information derived in the deriving unit c).

The method of the present invention includes step A) of constructing a system for providing mental illness-related information and drug prescription-related information, wherein the system includes: a) a network information providing unit in which a network derivable from mental illness-related questionnaire information is constructed; b) a clustering unit configured to classify mental illness groups based on mental illness-related questionnaire-based information and algorithm information based on the network information derived in a); and c) a drug prescription information deriving unit configured to derive network-based drug prescription information based on the network information derived in a) and cluster-based drug prescription information based on mental illness group information classified in b).

Specifically, step A) is a step of constructing a system for providing mental illness-related information and drug prescription-related information, wherein the system may include: a configuration that derives network information; a configuration that provides mental illness-related information by classifying mental illness groups based on clustering information; a configuration that derives cluster-based drug prescription information; and a configuration that derives drug prescription information based on the network information. More specifically, the system may include: a) a network information providing unit in which a network derivable from mental illness-related questionnaire information is constructed; b) a clustering unit configured to classify mental illness groups based on mental illness-related questionnaire-based information and algorithm information based on the network information derived in a); and c) a drug prescription information deriving unit configured to derive network-based drug prescription information based on the network information derived in a) and cluster-based drug prescription information based on mental illness group information classified in b).

The "mental illness-related information" is information that helps diagnose mental illness and does not mean the diagnosis of mental illness itself by a medical professional, and the mental illness-related information derived in the present invention may be information on a known mental illness. In addition, the "drug prescription-related information" is information that helps prescribe drugs for treating a diagnosed mental illness and does not mean the drug prescription itself by a medical professional, and the drug prescription-related information derived in the present invention may be information on a known drug prescription.

The network information providing unit a) is a configuration in which a network derivable from mental illness-related questionnaire information is constructed.

The "questionnaire" refers to a publicly known mental illness questionnaire, a questionnaire for diagnosis, and may be a publicly known questionnaire or specifically a UKB-based questionnaire.

The network information is information used for deriving network-based drug prescription information and the Louvain algorithm described below, and may be preconfigured based on mental illness-related questionnaire information before the patient's questionnaire information is provided.

The clustering unit b) is a configuration that classifies mental illness groups based on the mental illness-related questionnaire-based information and the network information-based algorithm information derived in a). The "clustering" means classifying based on the contents and/or results of the questionnaire.

In one embodiment of the present invention, the b) clustering unit may classify mental illness groups based on k-means and Louvain algorithms.

The k-means algorithm is an algorithm that classifies data into K clusters. The k-means algorithm proceeds in the following steps: setting the number of clusters (K); setting random initial centroids (K); assigning all data to the closest cluster; resetting the centroids using the data belonging to the cluster; reassigning data to clusters based on the new centroids; and repeating until there is no more movement of centroids. The Louvain algorithm is a type of network-based community detection method, and is similar to clustering in that it detects groups with high connection density, i.e. communities, on a network. The Louvain algorithm is a method introduced in 2008 and is an effective algorithm that utilizes the modularity and hierarchical structure of the network (Blondel V, Guillaume J-L, Lambiotte R, al. e. Fast unfolding of communities in large networks. Journal of Statistical Mechanics: Theory and Experiment. 2008;P10008).

The silhouette score was used to determine the optimal number of clusters. The silhouette score indicates how efficiently clusters are separated from each other and has a value between 0 and 1. The higher the cohesion within a cluster and the higher the separation between clusters, the closer the silhouette score is to 1.

In one embodiment of the present invention, clustering based on the k-means algorithm may be classification into 9 groups, and clustering based on the Louvain algorithm may classification into 10 groups.

c) is a configuration that derives network-based drug prescription information based on the network information derived in a) and cluster-based drug prescription information based on the mental illness group information classified in b), thereby providing both network-based drug prescription information and cluster-based drug prescription information.

The network-based drug prescription information is based on the network information derived in a), and is configured separately from the mental illness group classification by the above-described clustering unit b).

Specifically, the network-based drug prescription information is drug prescription information derived based on a list of drugs most likely to be prescribed to each person, created considering their neighbor's prescription history from the network generated for community detection in patient clustering. The network-based drug prescription information may be derived, for example, through steps of: (i) setting up an initial network composed of the nearest neighbors in a pre-constructed network (configuration (a)) of the system in the present invention); (ii) expanding the network to have at least N neighbors with a drug prescription history; and (iii) recommending a list of drugs sorted by frequency of drug treatment in the network.

The cluster-based drug prescription information is based on the mental illness group information classified in b) above.

The cluster-based drug prescription information is drug prescription information based on clusters distinguished based on the aforementioned k-means and Louvain algorithms, and includes not only drug prescription information common between the drug prescription information based on k-means algorithm and the drug prescription information based on the Louvain algorithm, but also drug prescription information of either one of them.

As an exemplary method of classifying groups in the clustering unit b) and deriving cluster-based drug prescription information in c), in the clustering unit b), groups were classified through the k-means algorithm based on questionnaire data from respondents in UKB cohort, with objects belonging to the cluster with each cluster's centroid, and groups were classified through the Louvain algorithm based on the network information derived in a) using 141 questionnaire items, age, and gender as data, and cluster-based drug prescription information in c) was derived by calculating the frequency of use of each drug based on information of each group classified in the clustering unit b), and then deriving the frequencies for four classes (AD, AP, MS, and SH).

The method of the present invention includes, after step A), step B) of inputting patient's questionnaire response information into the system and providing both mental illness-related information based on the mental illness groups classified in the clustering unit b) and drug prescription-related information derived in the deriving unit c).

Step B) is a step of inputting actual patient questionnaire information into the system constructed in step A) and providing mental illness-related information and drug prescription information derived through each of the configurations a), b), and c) of the system.

The information provided in step B) does not directly mean a diagnosis of mental illness or a prescription of medication as described above, but rather means information to assist a medical professional in making a judgment on the diagnosis of mental illness or the prescription of medication.

Another aspect of the present invention provides a system for providing mental illness-related information and drug prescription-related information, including: a network information providing unit in which a network derivable from mental illness-related questionnaire information is constructed; a clustering unit configured to classify mental illness groups based on mental illness-related questionnaire-based information and algorithm information based on the network information derived in the information providing unit; a cluster-based drug prescription information deriving unit configured to derive cluster-based drug prescription information based on mental illness group information classified in the clustering unit; a network-based drug prescription information deriving unit configured to derive network-based drug prescription information based on network information derived in the information providing unit; and an output unit configured to provide mental illness group information derived in the clustering unit and drug prescription information derived in the network-based drug prescription information deriving unit and the cluster-based drug prescription information deriving unit.

The system is configured to drive the above-described method. As described above, the network information providing unit may provide information related to the constructed network derivable from mental illness-related questionnaire information, and the clustering unit may classify mental illness groups based on k-means information based on the mental illness-related questionnaire and Louvain algorithm information based on the network information derived in the information providing unit.

In addition, the system includes: a cluster-based drug prescription information deriving unit configured to derive cluster-based drug prescription information based on mental illness group information classified in the clustering unit; a network-based drug prescription information deriving unit configured to derive network-based drug prescription information based on network information derived in the information deriving unit; and an output unit configured to provide mental illness group information derived in the clustering unit and drug prescription information derived in the network-based drug prescription information deriving unit and the cluster-based drug prescription information deriving unit.

Still another aspect of the present invention may provide a computer-readable recording medium having recorded thereon a program for executing the method or the system.

The method may be implemented in the form of program instructions that may be executed through various computer means and recorded on a computer-readable recording medium. Here, the recording medium may include program instructions, data files, data structures, and the like alone or in combination. The program instructions recorded on the recording medium may be those specially designed and configured for the above-described method or may be those known and available to those skilled in the field of computer software.

Examples of the recording medium include magnetic media such as a hard disk, a floppy disk, and a magnetic tape, optical media such as a CDROM (Compact Disk Read Only Memory) and a DVD (Digital Video Disk), magneto-optical media such as a floptical disk, and hardware devices specifically configured to store and execute program instructions, such as a ROM, a RAM (Random Access Memory), a flash memory, etc. Examples of program instructions include not only machine language codes such as those produced by a compiler, but also high-level language codes that may be executed by a computer using an interpreter, etc. Such hardware devices may be configured to operate as one or more software modules to perform the operations of the above-described method, and vice versa.

Implementations of the subject matter described in this specification may be implemented as one or more computer program products, i.e., one or more modules of computer program instructions encoded on a tangible program carrier for execution by, or to control the operation of, the apparatus according to the method. The computer-readable medium may be a machine-readable storage device, a machine-readable storage substrate, a memory device, a composition of matter affecting a machine-readable propagated signal, or a combination of one or more of them.

A computer program (also known as a program, software, software application, script, or code) mounted in an apparatus according to the above method and executing the above method may be written in any form of programming language, including compiled or interpreted languages, or declarative or procedural languages, and it may be deployed in any form, including as a stand-alone program or as a module, component, subroutine, or other unit suitable for use in a computing environment. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program may be deployed to be executed on one computer or on multiple computers that are located at one site or distributed across multiple sites and interconnected by a communication network.

### Mode for Invention

Hereinafter, one or more embodiments will be described in more detail by way of examples. However, these examples are intended to illustrate one or more embodiments, and the scope of the present invention is not limited to these examples.

### Example 1: Method for Providing Mental Illness-Related Information and Drug Prescription-Related Information

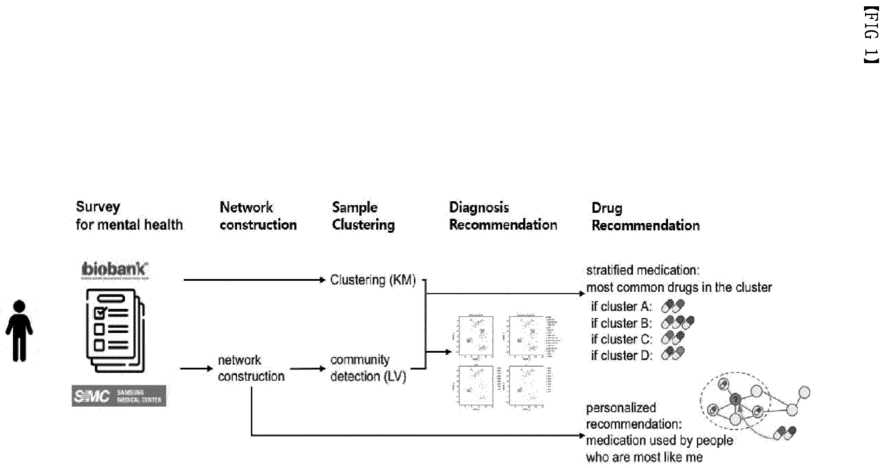

The method for providing information related to mental illness diagnosis and drug prescription according to the present invention is as follows. The general flow chart of the present invention is as shown in FIG. 1.

### Example 2: Method for Providing Mental Illness-Related Information and Drug Prescription-Related Information Using Information on UK Biobank Cohort

### 2-1. Mental Illness-Related Questionnaire Survey

The UK Biobank (UKB) is a prospective cohort of over 500,000 participants providing information regarding health status, lifestyle behavior, family history and sociodemographics. For mental health research, an online-based questionnaire was developed by an expert working group funded by UKB, and data was collected from 2016 to 2017 from participants who received an email invitation or were surveyed via a participant website. Detailed information about the questionnaire is available on the UKB website. The developed questionnaire covers life-time experiences in the area of mental health and addresses the following topics: (A) diagnostic screening, (B) mood (depression and bipolar), (C) anxiety, (D) general/drug addiction, (E) alcohol and cannabis use, (F) unusual and psychotic experiences, (G) traumatic events in life, and (H) harm behaviors. In the present invention, 141 questions from the developed questionnaire were used, as shown in Table 1 below.

**[Table 1]**

| Question category | Number |
|---|---|
| A. Mental distress (Dx Screening) | 3 |
| B. Mood: Depression/Mania | 37 |
| C. Anxiety | 28 |
| D. Addictions | 12 |
| E. Alcohol/Cannabis use | 11 |
| F. Unusual and psychotic experiences | 14 |
| G. Traumatic events | 21 |
| H. Self-harm behaviors | 10 |
| J. Happiness and subjective well-being | 3 |
| Total | 141 |

### 2-2. Network Construction Process

Through the questionnaire survey, the sample's characteristics used in the present invention are shown in Table 2 below.

**[Table 2]**

| | All | Female | Male |
|---|---|---|---|
| N | 157,348 (100.0%) | 89,089 (56.2%) | 58,259 (43.38%) |
| Age (mean, std) | 38-72 (55.93, 7.74) | 40-70 (55.45, 7.66) | 38-72 (56.56, 7.8) |

| **Self-reported Dx for mental disorders** | | | |
|---|---|---|---|
| Depression | 33,418 (21.24%) | 22,737 (25.52%) | 10,681 (15.65%) |
| Anxiety, nervousness, or generalized anxiety disorder | 22,033 (14.0%) | 14,760 (16.57%) | 7,273 (10.66%) |
| Panic attacks | 8,704 (5.53%) | 6,030 (6.77%) | 2,674 (3.92%) |
| Any other phobia (e.g., disabling fear of heights or spiders) | 2,153 (1.37%) | 1,488 (1.67%) | 665 (0.97%) |
| Social anxiety or social phobia | 1,962 (1.25%) | 1,060 (1.19%) | 902 (1.32%) |
| Obsessive compulsive disorder | 982 (0.62%) | 591 (0.66%) | 391 (0.57%) |
| Anorexia nervosa | 891 (0.57%) | 849 (0.95%) | 42 (0.06%) |
| Mania, hypomania, bipolar or manic-depression | 837 (0.53%) | 447 (0.5%) | 390 (0.57%) |
| Psychological over-eating or binge-eating | 706 (0.45%) | 600 (0.67%) | 106 (0.16%) |
| Any other type of psychosis or psychotic illness | 604 (0.38%) | 364 (0.41%) | 240 (0.35%) |
| Agoraphobia | 599 (0.38%) | 468 (0.53%) | 131 (0.19%) |
| Bulimia nervosa | 503 (0.32%) | 482 (0.54%) | 21 (0.03%) |
| Personality disorder | 385 (0.24%) | 185 (0.21%) | 200 (0.29%) |
| Autism, Asperger's, or autistic spectrum disorder | 223 (0.14%) | 67 (0.08%) | 156 (0.23%) |
| Schizophrenia | 157 (0.1%) | 54 (0.06%) | 103 (0.15%) |
| Attention deficit or attention deficit and hyperactivity disorder | 133 (0.08%) | 66 (0.07%) | 67 0.1%) |

Two types of diagnostic (Dx) information were used to construct the network. First, self-reported diagnosis (UKB field ID #20544, self-reported Dx) was used as reference. In addition, as shown in Table 3 below, the present inventors used score-based diagnosis (Dx) by modifying criteria from a comparative study of four different indicators of psychiatric disorders using UKB in 2019. In the present invention, network information was derived based on data obtained from 157,348 responders.

**[Table 3]**

| Diagnosis | Symptom | Criteria |
|---|---|---|
| Depression | Case: Depression ever. At least one core symptom of depression, for most of the day or during the whole day, on most or all days for a two week period, with at least five depressive symptoms that represent a change from the usual state over the same time period, with some or significant impairment. | -Persistent sadness (20446) = Yes OR Loss of interest (20441) = Yes |
| | | -How much of day (20436) = Most of day or All day long (3 or 4) |
| | | -Did you feel this way (20439) = Almost every day or Every day (2 or 3) |
| | | - Impairment (20440) = Somewhat or A lot (2 or 3) |
| | | - Total number of symptoms endorsed (core and others) >= 5 |
| | | Persistent sadness (core) 20446; Loss of interest (core) 20441; Tired or low energy 20449; Gain or loss of weight 20536 (1,2,3) = Gain, Loss, or Gain and loss; Sleep change 20532; Trouble concentrating 20435; Feeling worthless 20450; Thinking about death 20437 |
| Anxiety disorder | Case: GAD Ever. Excessive worrying regarding numerous issues, occurring on most days for six months, and that are difficult to control, with three or more somatic symptoms and functional impairment. | - Worried, tense, or anxious (20421) = Yes |
| | | - Duration (20420) >= 6 months or All my life (;-999) |
| | | - Most days (20538) = Yes |
| | | - Excessive: More than most (20425) OR Stronger than most (20542) |
| | | - Number of issues: More than one thing (20543; 2) OR Different worries (20540) |
| | | - Difficult to control: Difficult to stop worrying (20541) OR Couldn't put it out of mind (20539; 3) OR Difficult to control (20537; 3) |
| | | - Functional impairment: Role interference (20418) = Some or A lot (2,3) |
| | | - 3 somatic symptoms out of: Restless. 20426; Keyed up or on edge. 20423; Easily tired. 20429; Having difficulty keeping your mind on what you were doing. 20419; More irritable than usual. 20422; Having tense, sore, or aching muscles. 20417; Frequently having trouble falling or staying asleep. 20427 |
| Bipolar disorder | Symptoms: Hypomania / Mania. Endorses features of hypomania / mania lasting for a week or more, whether or not they are disruptive, and with or without a history of depression. Requires "mania" plus three other symptoms or "Irritable" plus four other symptoms. | - High/Hyper 20501 = 01 OR Irritable 20502 = 01 |
| | | - Four features from: High/Hyper 20501; Active 20548(01); Talkative 20548(02); Less sleep 20548(03); Creative/ideas 20548(04); Restless 20548(5); Confident 20548(6); Thoughts racing 20548(7); Easily distracted 20548(8) |
| | | - Duration 20492 = A week or more (3) |
| Psychotic disorder including schizophrenia | Symptoms: Psychotic experience. Endorsed possible hallucination or delusion | - Heard unreal voice 20463 = yes |
| | | - Saw unreal vision 20471 = yes |
| | | - Believed unreal conspiracy 20468 = yes |
| | | - Believed unreal communication or signs 20474 = yes |

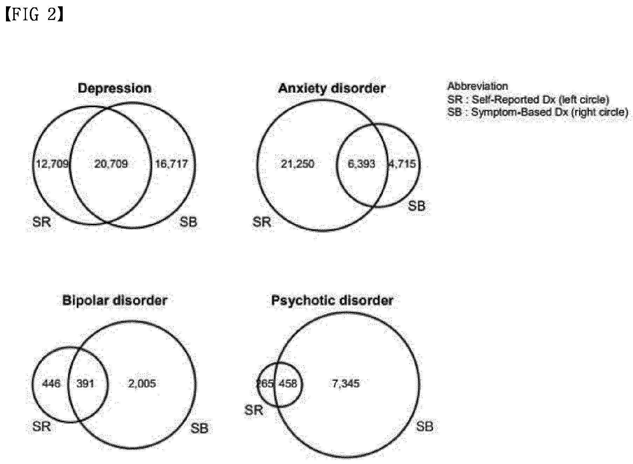

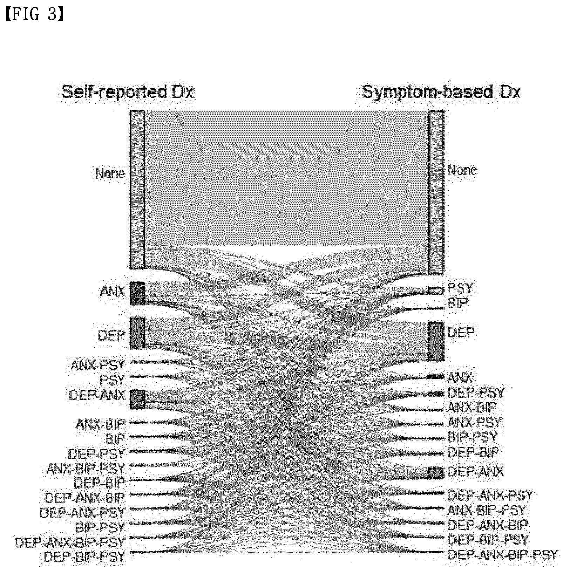

As shown in Tables 4 and 5 below and FIGS. 2 and 3, as a result of comparing the prevalence of mental illnesses between self-reported diagnosis (Dx) and symptom-based diagnosis (Dx), inconsistencies were detected in 33.28% (52,366) of participants.

**[Table 4]**

| | Self-reported Dx (SR) | Symptom-based Dx (SB) | Overlap |
|---|---|---|---|
| Depression | 33,418 (21.24%) | 37,426 (23.79%) | 20,709 (13.16%) |
| Anxiety disorder | 27,643 (17.57%) | 11,108 (7.06%) | 6,393 (4.06%) |
| Bipolar disorder | 837 (0.53%) | 2,396 (1.52%) | 391 (0.25%) |
| Psychotic disorder | 723 (0.46%) | 7,803 (4.96%) | 458 (0.29%) |

**[Table 5]**

| | Self-reported Dx | Symptom-based Dx |
|---|---|---|
| None | 108,451 (68.92%) | 112,757 (71.66%) |
| DEP | 20,547 (13.06%) | 25,957 (16.50%) |
| DEP-ANX | 12,002 (7.63%) | 6,717 (4.27%) |
| ANX | 15,011 (9.54%) | 2,316 (1.47%) |
| PSY | 97 (0.06%) | 3,768 (2.39%) |
| DEP-PSY | 123 (0.08%) | 2,140 (1.36%) |
| DEP-ANX-PSY | 223 (0.14%) | 1,062 (0.67%) |
| DEP-BIP | 165 (0.10%) | 668 (0.42%) |
| BIP | 205 (0.13%) | 606 (0.39%) |
| DEP-ANX-BIP | 214 (0.14%) | 435 (0.28%) |
| DEP-ANX-BIP-PSY | 96 (0.06%) | 227 (0.14%) |
| ANX-PSY | 57 (0.04%) | 235 (0.15%) |
| DEP-BIP-PSY | 48 (0.03%) | 220 (0.14%) |
| BIP-PSY | 69 (0.04%) | 124 (0.08%) |
| ANX-BIP | 30 (0.02%) | 89 (0.06%) |
| ANX-BIP-PSY | 10 (0.01%) | 27 0.02%) |

Through the foregoing, it was confirmed that diagnosis was not easy because the results were different between self-reported diagnosis and symptom-based diagnosis. Therefore, in the present invention, a network was constructed using the questionnaire survey results and clustering was performed.

In addition, in the present invention, web-based questionnaire data on mental health from 157,348 responders, used in derivation of the network information, were used for clustering analysis. In addition, data on medication use based on self-report - available from 14,358 respondents (UKB field id #20003)- were used for drug recommendation. As shown in Tables 6 and 7 below, after pre-processing, including the correction of typos and removal of duplicates, 154 psychotropic drugs were selected through manual curation by a psychiatrist, and classified into four different drug classes, including antidepressant (AD), anti-psychotic (AP) medication, mood stabilizer (MS), and sedative-hypnotic (SH) drug.

**[Table 6]**

| Drug class (curated by clinician) | Number (N) |
|---|---|
| AD: Antidepressant | 55 |
| AP: Antipsychotics | 44 |
| MS: Mood stabilizer | 13 |
| SH: Sedative hypnotic | 26 |
| Etc | 16 |
| Total | 154 |

**[Table 7]**

| No | Drug class | Drug name | UKB code |
|---|---|---|---|
| 1 | AD | citalopram | 1140921600 |
| 2 | AD | fluoxetine | 1140879540 |
| 3 | AD | amitriptyline | 1140879616 |
| 4 | AD | sertraline | 1140867878 |
| 5 | AD | venlafaxine | 1140916282 |
| 6 | AD | paroxetine | 1140867888 |
| 7 | AD | Mirtazapine | 1141152732 |
| 8 | AD | dosulepin | 1140909806 |
| 9 | AD | escitalopram | 1141180212 |
| 10 | AD | seroxat 20mg tablet | 1140882236 |
| 11 | AD | prozac 20mg capsule | 1140867876 |
| 12 | AD | trazodone | 1140879634 |
| 13 | AD | st john's wort/hypericum [ctsu] | 1201 |
| 14 | AD | Duloxetine | 1141200564 |
| 15 | AD | cipralex 5mg tablet | 1141190158 |
| 16 | AD | lofepramine | 1140867726 |
| 17 | AD | clomipramine | 1140879620 |
| 18 | AD | efexor 37.5mg tablet | 1140916288 |
| 19 | AD | nortriptyline | 1140867818 |
| 20 | AD | imipramine | 1140879630 |
| 21 | AD | cipramil 10mg tablet | 1141151946 |
| 22 | AD | dothiepin | 1140879628 |
| 23 | AD | prothiaden 25mg capsule | 1140867624 |
| 24 | AD | reboxetine | 1141151978 |
| 25 | AD | lustral 50mg tablet | 1140867884 |
| 26 | AD | trimipramine | 1140867756 |
| 27 | AD | zispin 30mg tablet | 1141152736 |
| 28 | AD | amitriptyline hydrochloride + perphenazine 10mg/2mg tablet | 11410867948 |
| 29 | AD | cymbalta 30mg gastro-resistant capsule | 1141201834 |
| 30 | AD | anafranil 10mg capsule | 1140867690 |
| 31 | AD | moclobemide | 1140867920 |
| 32 | AD | phenelzine | 1140867850 |
| 33 | AD | fluvoxamine | 1140879544 |
| 34 | AD | tranylcypromine | 1140867914 |
| 35 | AD | surmontil 10mg tablet | 1140867758 |
| 36 | AD | doxepin | 1140867640 |
| 37 | AD | triptafen tablet | 1140867934 |
| 38 | AD | yentreve 20mg gastro-resistant capsule | 1141200570 |
| 39 | AD | nardil 15mg tablet | 1140867852 |
| 40 | AD | edronax 4mg tablet | 1141151982 |
| 41 | AD | allegron 10mg tablet | 1140867820 |
| 42 | AD | Mianserin | 1140879556 |
| 43 | AD | faverin 50mg tablet | 1140867860 |
| 44 | AD | tranylcypromine+trifluoperazine 10mg/1mg tablet | 1140867944 |
| 45 | AD | molipaxin 50mg capsule | 1140882244 |
| 46 | AD | Nefazodone | 1140917460 |
| 47 | AD | gamanil 70mg tablet | 1140882310 |
| 48 | AD | Amitriptyline + chlordiazepoxide 12.5mg/5mg capsule | 1140867938 |
| 49 | AD | manerix 150mg tablet | 1140867922 |
| 50 | AD | maoi - tranylcypromine | 1140910820 |
| 51 | AD | limbitrol 10 capsule | 1140856186 |
| 52 | AD | norval 10mg tablet | 1140867812 |
| 53 | AD | tofranil 10mg tablet | 1140867712 |
| 54 | AD | Amoxapine | 1140867774 |
| 55 | AD | sinequan 10mg capsule | 1140882312 |
| 56 | AP | olanzapine | 1140928916 |
| 57 | AP | quetiapine | 1141152848 |
| 58 | AP | risperidone | 1140867444 |
| 59 | AP | chlorpromazine | 1140879658 |
| 60 | AP | trifluoperazine | 1140868120 |
| 61 | AP | amisulpride | 1141153490 |
| 62 | AP | seroquel 25mg tablet | 1141152860 |
| 63 | AP | sulpiride | 1140867304 |
| 64 | AP | aripiprazole | 1141195974 |
| 65 | AP | haloperidol | 1140867168 |
| 66 | AP | stelazine 1 mg tablet | 1140867244 |
| 67 | AP | depixol 3mg tablet | 1140867152 |
| 68 | AP | clozapine | 1140867420 |
| 69 | AP | Flupentixol | 1140909800 |
| 70 | AP | Promazine | 1140879746 |
| 71 | AP | fluanxol 500micrograms tablet | 1140867952 |
| 72 | AP | risperdal 0.5mg tablet | 1141177762 |
| 73 | AP | modecate 12.5mg/0.5ml oily injection | 1140867456 |
| 74 | AP | zyprexa 2.5mg tablet | 1141167976 |
| 75 | AP | flupenthixol | 1140867150 |
| 76 | AP | zuclopenthixol | 1140882100 |
| 77 | AP | largactil 10mg tablet | 1140863416 |
| 78 | AP | haldol 5mg tablet | 1140867184 |
| 79 | AP | abilify 5mg tablet | 1141202024 |
| 80 | AP | clopixol 2mg tablet | 1140867342 |
| 81 | AP | clozaril 25mg tablet | 1140882320 |
| 82 | AP | cpz - chlorpromazine | 1140910358 |
| 83 | AP | fluphenazine | 1140882098 |
| 84 | AP | pericyazine | 1140867134 |
| 85 | AP | fentazin 2mg tablet | 1140867210 |
| 86 | AP | fluphenazine decanoate | 1140867398 |
| 87 | AP | dolmatil 200mg tablet | 1140867306 |
| 88 | AP | levomepromazine | 1140909802 |
| 89 | AP | perphenazine | 1140867208 |
| 90 | AP | zaponex 25mg tablet | 1141201792 |
| 91 | AP | neulactil 2.5mg tablet | 1140867136 |
| 92 | AP | Zotepine | 1141169714 |
| 93 | AP | serenace 500micrograms capsule | 1140867092 |
| 94 | AP | Thioridazine | 1140879750 |
| 95 | AP | denzapine 25mg tablet | 1141200458 |
| 96 | AP | pimozide | 1140867218 |
| 97 | AP | fluphenazine hydrochloride + nortriptyline 1.5mg/30mg tablet | 1140867940 |
| 98 | AP | benperidol | 1140867078 |
| 99 | AP | sertindole | 1140927956 |
| 100 | MS | lithium product | 1140867490 |
| 101 | MS | priadel 200mg m/r tablet | 1140867504 |
| 102 | MS | Lamotrigine | 1140872290 |
| 103 | MS | tegretol 100mg tablet | 1140872072 |
| 104 | MS | sodium valproate | 1140872198 |
| 105 | MS | Carbamazepine | 2038459704 |
| 106 | MS | epilim 100mg crushable tablet | 1140872200 |
| 107 | MS | depakote 250mg e/c tablet | 1141172838 |
| 108 | MS | Topiramate | 1140923484 |
| 109 | MS | valproic acid | 1140872214 |
| 110 | MS | lamictal 25mg tablet | 1140872302 |
| 111 | MS | camcolit 250 tablet | 1140867494 |
| 112 | MS | carbamazepine product | 1140872064 |
| 113 | SH | zopiclone | 1140863144 |
| 114 | SH | diazepam | 1140863152 |
| 115 | SH | temazepam | 1140863202 |
| 116 | SH | zolpidem | 1140865016 |
| 117 | SH | clonazepam | 1140872150 |
| 118 | SH | nitrazepam | 1140863182 |
| 119 | SH | Lorazepam | 1140863302 |
| 120 | SH | zimovane ls 3.75mg tablet | 1140928004 |
| 121 | SH | valium 2mg tablet | 1140863244 |
| 122 | SH | Oxazepam | 1140863442 |
| 123 | SH | chlordiazepoxide | 1140863328 |
| 124 | SH | Clobazam | 1140863268 |
| 125 | SH | Loprazolam | 1140863120 |
| 126 | SH | stilnoct 5mg tablet | 1140864916 |
| 127 | SH | xanax 250mcg tablet | 1140863310 |
| 128 | SH | alprazolam | 1140863308 |
| 129 | SH | diazepam product | 1141157496 |
| 130 | SH | Zaleplon | 1141171404 |
| 131 | SH | sonata 5mg capsule | 1141171410 |
| 132 | SH | Bromazepam | 1140863318 |
| 133 | SH | ativan 1mg tablet | 1140863364 |
| 134 | SH | Medazepam | 1140863372 |
| 135 | SH | valium 10mg suppository | 1140855856 |
| 136 | SH | mogadon 5mg tablet | 1140863194 |
| 137 | SH | rohypnol 1 mg tablet | 1140863106 |
| 138 | SH | flurazepam | 1140863110 |
| 139 | etc | propranolol | 1140879842 |
| 140 | etc | gabapentin | 1140872228 |
| 141 | etc | pregabalin | 1141200004 |
| 142 | etc | procyclidine | 1140883476 |
| 143 | etc | clonidine | 1140883468 |
| 144 | etc | ropinirole | 1140928274 |
| 145 | etc | nicotine product | 1140872492 |
| 146 | etc | buspirone | 1140879730 |
| 147 | etc | inderal 10mg tablet | 1140866804 |
| 148 | etc | clonidine hydrochloride 25micrograms tablet | 1140871986 |
| 149 | etc | trihexyphenidyl | 1140909816 |
| 150 | etc | bupropion | 1141176854 |
| 151 | etc | buspar 5mg tablet | 1140863454 |
| 152 | etc | donepezil hydrochloride | 1141150834 |
| 153 | etc | atomoxetine | 1141199446 |
| 154 | Etc | atomoxetine | 1141167690 |

For network-based community detection, the present inventors first constructed a network using the k-nearest neighbor algorithm, wherein nodes represented each sample and edges indicated similarity between samples based on symptoms and history of mental illness. The present inventors took 100 nearest neighbors for each node, thereby constructing a network.

### 2-3. Clustering Analysis

The present inventors performed clustering analysis based on questionnaire data from 157,348 respondents in UKB cohort. The clustering analyses were conducted using two different methods, that is, k-means clustering and network-based community detection. In k-means clustering, objects were divided into k clusters, with objects belonging to the cluster with each cluster's centroid.

In addition, for community detection, the Louvain algorithm was applied to the network constructed in 2-2 above. The Louvain algorithm optimized a network's modularity by repetitively performing community building within the network. Overall, 141 questionnaire items, age and gender were included for analyzing the data. The present inventors applied one-hot-encoding for categorical variables and removed ambiguous variables indicating missing values. Finally, 268 features were available for analysis. Standard scaling was applied for normalization.

The clustering analysis was implemented with changing number of clusters and resolution, for the k-means and the Louvain algorithm, respectively. To determine the optimal number of clusters, silhouette score was used. To visualize clustering results, the present inventors performed Uniform Manifold Approximation and Projection (UMAP), which is a nonlinear dimension reduction algorithm and an effective tool for visualizing clusters with their relative proximities.

The present inventors investigated clusters through three methods to confirm clinical significance. First, clustering results were mapped to diagnosis information, which were visualized using the Sankey diagram, to reveal their correlations through proportion of overlapping. Second, to investigate the questions that were most influential for each cluster, the present inventors performed LASSO (Least Absolute Shrinkage and Selection Operator) regression analysis. The present inventors identified questionnaire items as significant if they exhibited a beta coefficient greater than 0.2. Finally, the present inventors computed index scores for each category of questionnaire items (A) to (H) to examine each feature's importance. Category (J) among the index scores had a very small number of questions (3), and was excluded because it had no discriminatory power in the actual index score calculation. After normalizing data by min-max scaling, the present inventors summarized responses by averaging the values obtained for each category. Thereafter, the present inventors performed clustering analysis based on age, gender, and questionnaire data.

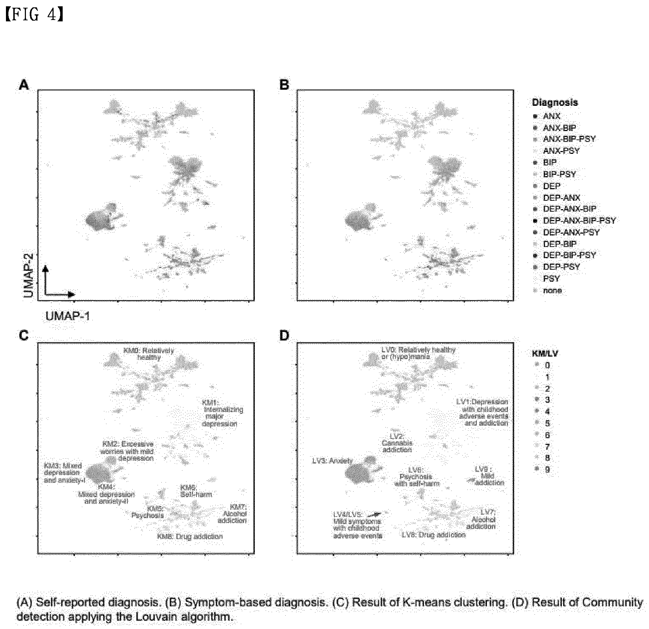

As shown in FIG. 4, nine and ten clusters were identified by k-means and Louvain methods, respectively.

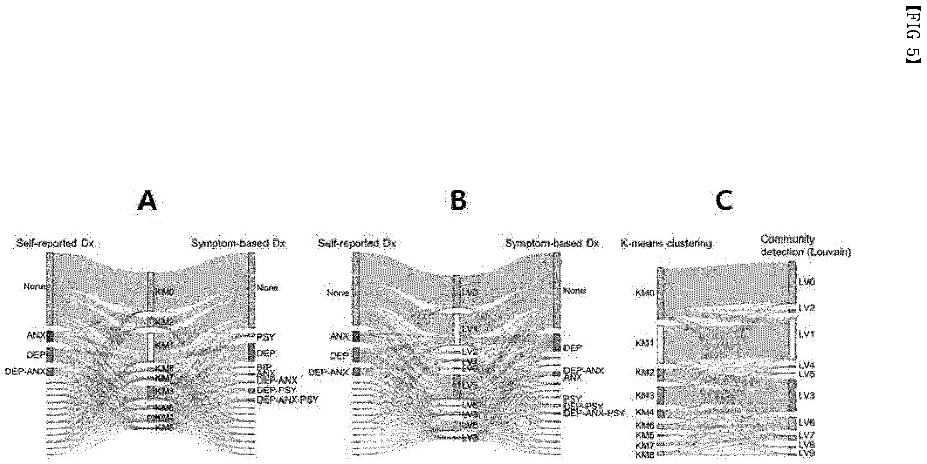

As shown in FIG. 5, the present inventors mapped clinical diagnostic information to the clustering results to visualize diagnostic components within each cluster. It was found that KM0 and LV0 predominantly comprised participants who were mentally healthy, while clusters KM1 and LV1 included participants who were relatively healthy to mildly depressed. Also, participants who presented both depression and anxiety symptoms were mostly assigned to KM2, KM3, KM4, and LV3. KM5, KM6, and LV6 included participants with multiple diagnoses and psychotic symptoms. Comparing KM and LV clustering, participants were most consistently assigned to KM0 and LV0, KM1 and LV1, and KM2-4 and LV3.

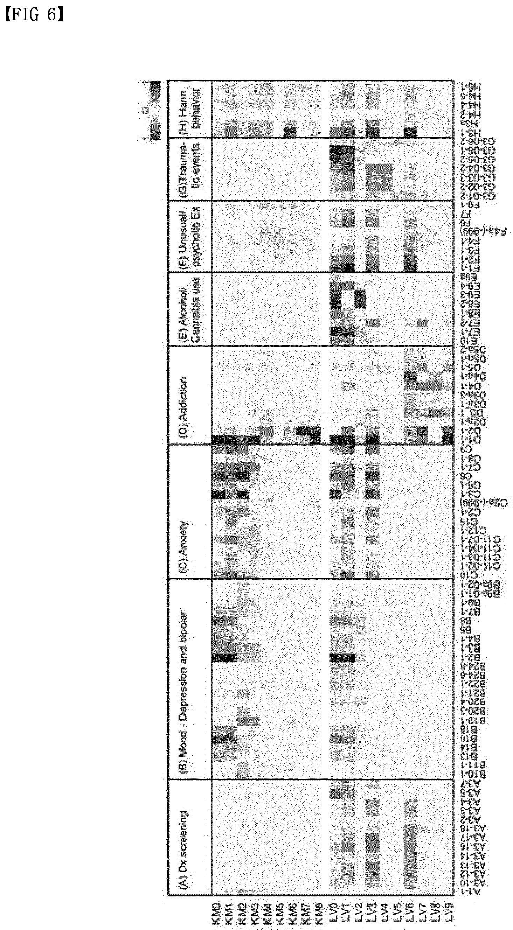

Subsequently, as shown in FIG. 6, the present inventors performed LASSO regression analysis to investigate feature importance for each cluster. Items exhibiting the highest feature importance were as follows: "ever depressed for two weeks or more in a row" from (B) depression in KM1 and LV1; "ever worried a lot more than most people would in your situation" from (C) anxiety in KM2, KM3, KM4, and LV3; "ever seen illusions" from (F) unusual experiences in KM5 and LV6; "deliberately harmed yourself" from (H) harm behavior in KM6 and LV6; "ever addicted to alcohol" from (E) alcohol/cannabis use in KM7 and LV7; "ever addicted to one or more things, including substances or behavior" from (D) general/drug addiction in KM8 and LV9; "how often did you take cannabis" from (E) alcohol/cannabis use in LV2; "diagnosed with a life-threatening illness" from (G) traumatic events in LV4; "involved in combat or exposed to a war-zone" from (G) traumatic events in LV5; and "ever addicted to prescription or over-the-counter medicine/ Illicit or recreational drugs" from (D) general/drug addiction in LV8. Further, the present inventors calculated scores by summarizing responses into eight categories of the questionnaire.

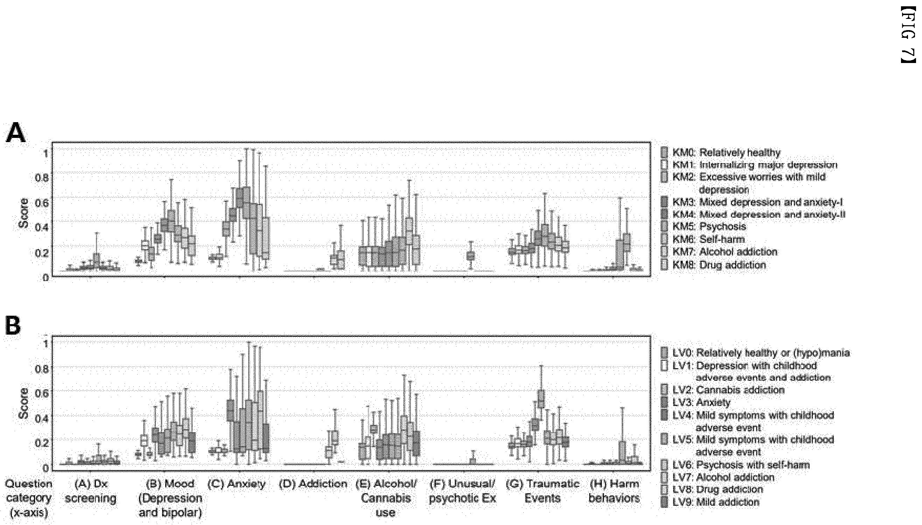

As shown in FIG. 7, the scores were considered as the indicator for the level of distinct characteristics for each category. Based on the average scores for each category, the result was predominantly consistent with the feature importance pattern for most clusters. The pattern of increasing scores representing anxiety symptoms was found from KM2 to KM3 to KM4.

Finally, considering the patterns of diagnostic mapping, feature importance, and scoring, the present inventors designated KM0 as "relatively healthy," KM1 as "internalizing major depression," KM2 as "excessive worries with mild depression," KM3 and KM4 as "mixed depression and anxiety," KM5 as "psychosis," KM6 as "self-harm," KM7 as "alcohol addiction," and KM8 as "drug addiction." For the LV result, the present inventors designated LV0 as "relatively healthy or (hypo)mania," LV1 as "depression with childhood adverse events and addiction," LV2 as "cannabis addiction," LV3 as "anxiety," LV4 and LV5 as "mild symptoms with childhood adverse events," LV6 as "psychosis with self-harm," LV7 as "alcohol addiction," LV8 as "drug addiction," and LV9 as "mild addiction."

### 2-4. Derivation of Drug Prescription Information

For drug recommendation, the present inventors developed two different types of methods for deriving drug prescription information.

The first type was the clustering-based recommendation. The present inventors calculated the frequency of use for each drug within each derived cluster and, thereafter, summarized the frequencies for four classes (AD, AP, MS, and SH). The resulting statistics indicate the likelihood of each drug class being prescribed to patients in each cluster.

The second type was network-based recommendation. From the network generated for community detection in patient clustering, the present inventors created a list of medications that each person was most likely to be prescribed, considering their neighbor's prescription history.

The steps of executing the network-based drug recommendation system were as follows: step (i) of finding nearest neighbors on a pre-constructed network; step (ii) of expanding the network to have at least N neighbors with a drug prescription history, and step (iii) of recommending a list of drugs sorted by frequency of drug treatment in the network. Here, the present inventors used the data of 14,358 individuals for which information on drug prescription was available. Recommendation was defined as the drug classes whose prescription ranking was higher than a predetermined threshold. Thereafter, the present inventors compared the predicted drug classes with the drug class reported to have been prescribed to each individual. Further, the present inventors calculated each drug class's recommendation frequency for each cluster.

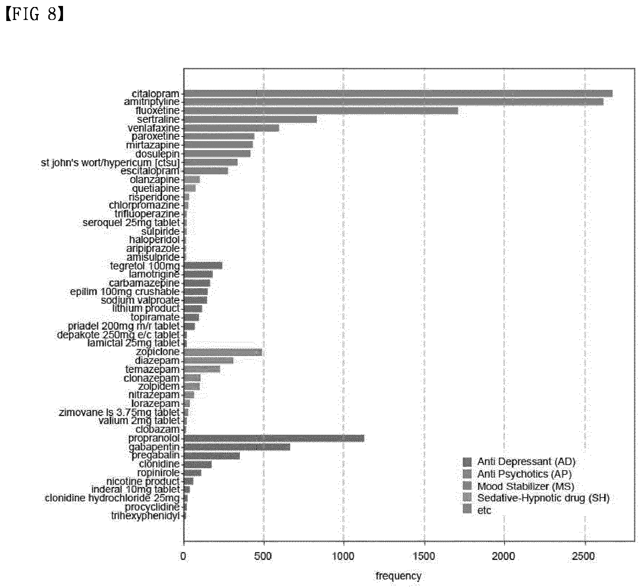

Information regarding drug prescription was available for 14,358 participants (9.12%) of the total sample, including 154 different psychotropic drugs. The most frequently prescribed drugs for each drug class are shown in FIG. 8.

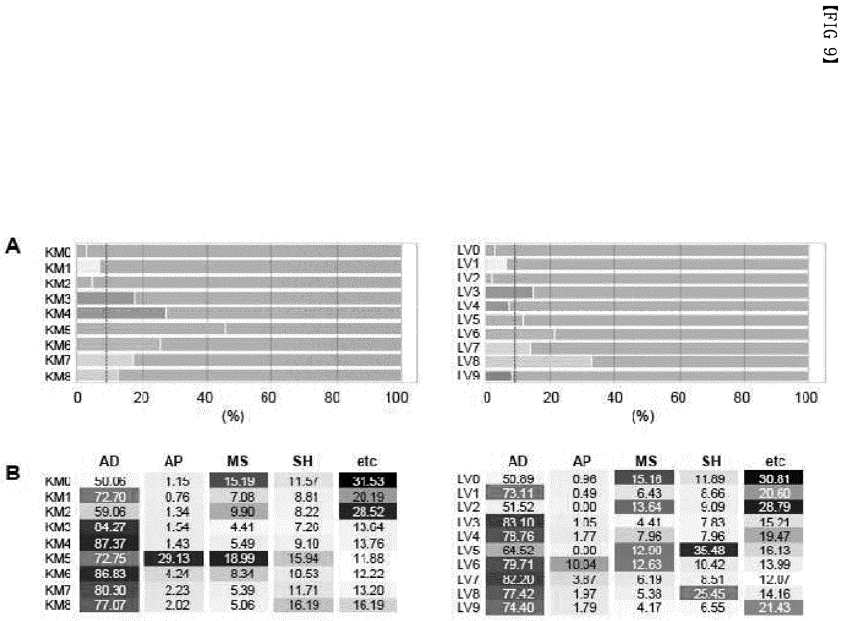

For cluster-based recommendation, the present inventors calculated the proportion of samples with drug prescription history in each cluster. As a result, as shown in FIG. 9A, KM5 exhibited the highest prescription rate (45.67%), followed by KM4 (27.35%) and KM6 (25.62%). In LV clustering, LV8 exhibited the highest prescription rate (32.69%), followed by LV6 (21.24%) and LV3 (14.84%). In addition, as shown in FIG. 9B, thereafter, the average prescription probability for each drug class was calculated and used for cluster-based recommendation. As shown therein, AD was commonly used across all clusters. Except for KM0, KM2, LV0, LV2, and LV5, the prescription probability of AD was over 70%. AP users were relatively aggregated in a single cluster of KM5 (29.13%) or LV6 (10.04%). This was in contrast to the prescription pattern of MS. In LV clustering, LV0 (15.15%) exhibited the highest rate of MS prescription, followed by LV2 (13.64%), LV5 (12.9%), and LV6 (12.63%). SH use was most common in KM8 (16.19%), KM5 (15.94%), LV5 (35.48%), and LV8 (25.45%).

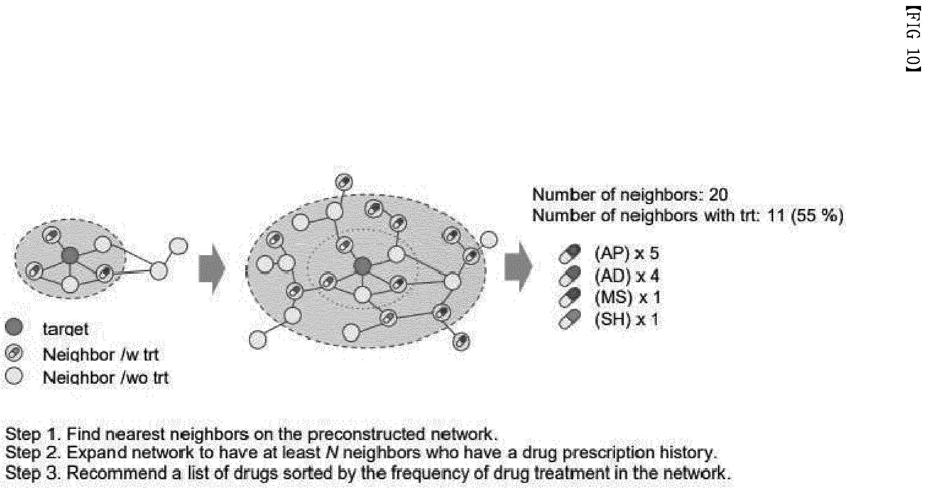

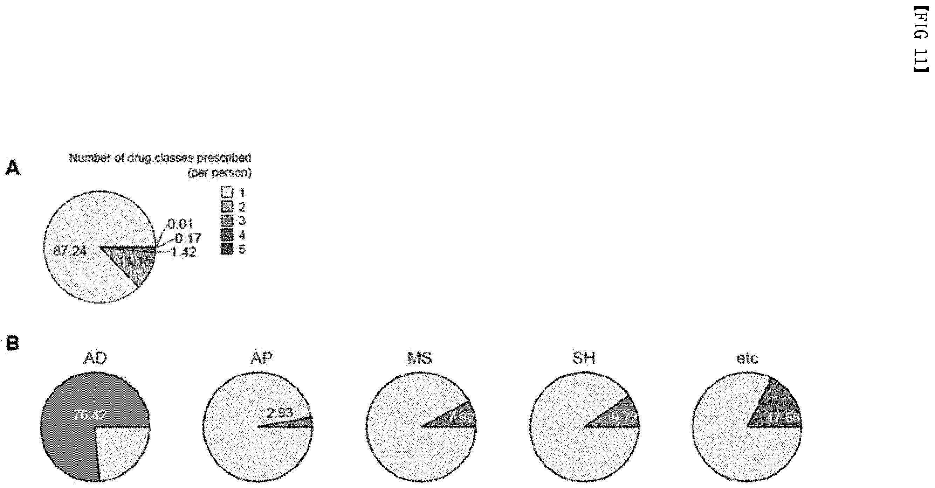

As shown in FIG. 10, the network-based drug recommendation was performed using drug prescription information of surrounding neighbors in the network to recommend drugs, and evaluation was performed in 14,358 individuals for whom information on drug use was available. As shown in FIG. 11A, among 14,358 individuals for whom information on drug use was available, 12,526 individuals (87.2%) had been prescribed with one of the drug classes, while 1,832 individuals (12.8%) received multiple drug classes. In addition, as shown in FIG. 11B, AD was used in 10,972 individuals (76.42%), AP in 421 individuals (2.93%), MS in 1,123 individuals (7.82%), and SH in 1,395 individuals (9.72%).

For the three most commonly recommended drugs, a single drug class was recommended to 6,211 individuals (43.26%), two drug classes to 5,876 individuals (40.92%), three drug classes to 1,619 individuals (11.28%), four drug classes to 497 individuals (3.46%), and five drug classes to 155 individuals (1.08%). Considering the different drug classes, AD drugs were recommended to 14,303 individuals (99.62%), AP drugs to 517 individuals (3.6%), MS drugs to 1,839 individuals (12.81%), and SH drugs to 1,744 individuals (12.15%).

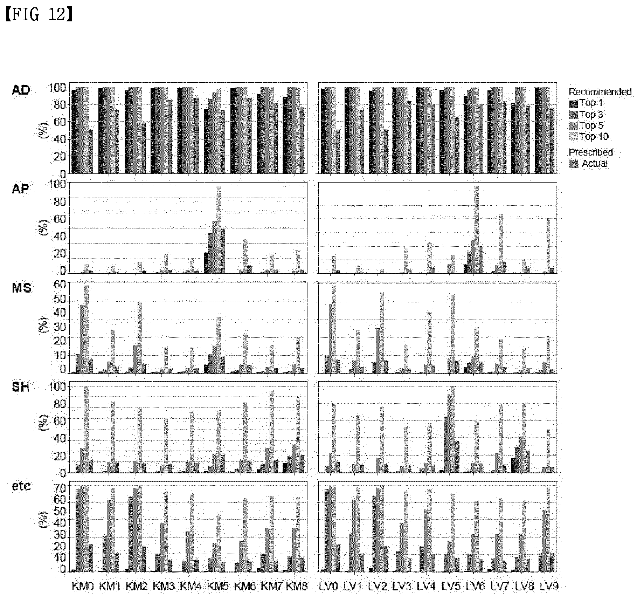

As shown in FIG. 12, when combining the network-based recommendation results with the cluster-based recommendation results, AD drugs were commonly recommended across all clusters. In addition, as in the actual prescription data, AP drugs were predominantly recommended to the individuals in KM5 and LV6. MS drugs were highly recommended for KM0, KM5, and LV0, but less so for LV5, compared to the actual treatment data. Also, SH drugs were predominantly recommended for KM8, LV5, and LV8.

### Example 3: Method of Providing Mental Illness-Related Information and Drug Prescription-Related Information Using Information of SMC Cohort

The method for providing mental illness-related information and drug prescription-related information was performed in the same manner as the method described in Example 2, but the cohort information was changed to the SMC cohort information.

### 3-1. Mental Illness-Related Questionnaire Survey

The Korean data comprised data from psychological evaluations as well as prescription information of 1,307 patients who visited a psychiatry department of Samsung Medical Center (SMC), a university-affiliated tertiary hospital in Seoul, Korea. Psychological evaluation data included 318 items from structural interviews, clinical rating scales, and self-reported scales. This psychological evaluation was named "Adult Screening Psychological evaluation." This evaluation battery, which lacks items for psychotic symptom's detailed evaluation, was mainly prescribed at the initial screening of patients exhibiting a low probability for psychosis. Of the respective items shown in Table 8 below, 80 questions with the lowest number of missing responses and 842 patients with no missing data were included in the analyses. The questionnaire data included 20 questions from a structural interview (M.I.N.I.), 33 questions from the clinician rating scales Hamilton depression rating scale (HAMD), Hamilton anxiety rating scale (HAMA), and Panic Disorder Severity Scale (PDSS), and 27 questions from self-reported scales.

**[Table 8]**

| No | Category | Section | Question | Q-category |
|---|---|---|---|---|
| 1 | M.I.N.I | Major Depressive Episode | Depressed mood (Last two weeks) | depression |
| 2 | M.I.N.I | Major Depressive Episode | Diminished interest or pleasure (Last two weeks) | depression |
| 3 | M.I.N.I | Major Depressive Episode | Weight loss or gain (Last two weeks) | depression |
| 4 | M.I.N.I | Major Depressive Episode | Insomnia or hypersomnia (Last two weeks) | depression |
| 5 | M.I.N.I | Major Depressive Episode | Psychomotor agitation or retardation (Last two weeks) | depression |
| 6 | M.I.N.I | Major Depressive Episode | Fatigue or loss of energy (Last two weeks) | depression |
| 7 | M.I.N.I | Major Depressive Episode | Feelings of worthlessness or excessive or inappropriate guilt (Last two weeks) | depression |
| 8 | M.I.N.I | Major Depressive Episode | Diminished ability to think or concentrate or indecisiveness (Last two weeks) | depression |
| 9 | M.I.N.I | Major Depressive Episode | Recurrent thoughts of death or suicidal ideation (Last two weeks) | harm |
| 10 | M.I.N.I | Major Depressive Episode | Number of symptoms (Last two weeks) | depression |
| 11 | M.I.N.I | Major Depressive Episode | Insomnia or hypersomnia (Current) | depression |
| 12 | M.I.N.I | Major Depressive Episode | Fatigue or loss of energy (Current) | depression |
| 13 | M.I.N.I | Major Depressive Episode | Diminished ability to think or concentrate or indecisiveness (Current) | depression |
| 14 | M.I.N.I | Major Depressive Episode | Recurrent thoughts of death or suicidal ideation (Current) | self-harm |
| 15 | M.I.N.I | Major Depressive Episode | Depressed mood (Past) | depression |
| 16 | M.I.N.I | Major Depressive Episode | Recurrent thoughts of death or suicidal ideation (Past) | self-harm |
| 17 | M.I.N.I | Suicidal Tendency | Recurrent thoughts of death (Lifetime) | self-harm |
| 18 | M.I.N.I | Suicidal Tendency | Suicidal ideation (Lifetime) | self-harm |
| 19 | M.I.N.I | Suicidal Tendency | Suicidal plan (Lifetime) | self-harm |
| 20 | M.I.N.I | Suicidal Tendency | Suicide attempt (Lifetime) | self-harm |
| 21 | Clinician Rating Scales | Hamilton Rating Scale for Depression | Depressed mood | depression |
| 22 | Clinician Rating Scales | Hamilton Rating Scale for Depression | Feelings of guilt | depression |
| 23 | Clinician Rating Scales | Hamilton Rating Scale for Depression | Suicide attempt (Lifetime) | self-harm |
| 24 | Clinician Rating Scales | Hamilton Rating Scale for Depression | Insomnia early | depression |
| 25 | Clinician Rating Scales | Hamilton Rating Scale for Depression | Insomnia middle | depression |
| 26 | Clinician Rating Scales | Hamilton Rating Scale for Depression | Insomnia late | depression |
| 27 | Clinician Rating Scales | Hamilton Rating Scale for Depression | Work and activities | depression |
| 28 | Clinician Rating Scales | Hamilton Rating Scale for Depression | Retardation | depression |
| 29 | Clinician Rating Scales | Hamilton Rating Scale for Depression | Agitation | anxiety |
| 30 | Clinician Rating Scales | Hamilton Rating Scale for Depression | Anxiety | anxiety |
| 31 | Clinician Rating Scales | Hamilton Rating Scale for Depression | Anxiety-somatic | anxiety |
| 32 | Clinician Rating Scales | Hamilton Rating Scale for Depression | Somatic symptoms | depression |
| 33 | Clinician Rating Scales | Hamilton Rating Scale for Depression | Somatic symptoms gastrointestinal | depression |
| 34 | Clinician Rating Scales | Hamilton Rating Scale for Depression | Genital symptoms | depression |
| 35 | Clinician Rating Scales | Hamilton Rating Scale for Depression | Hypochondriasis | anxiety |
| 36 | Clinician Rating Scales | Hamilton Rating Scale for Depression | Weight loss | depression |
| 37 | Clinician Rating Scales | Hamilton Rating Scale for Depression | Insight | depression |
| 38 | Clinician Rating Scales | Hamilton Rating Scale for Depression | Total score | depression |
| 39 | Clinician Rating Scales | Hamilton Rating Scale for Anxiety | Anxious mood | anxiety |
| 40 | Clinician Rating Scales | Hamilton Rating Scale for Anxiety | Tension | anxiety |
| 41 | Clinician Rating Scales | Hamilton Rating Scale for Anxiety | Fears | anxiety |
| 42 | Clinician Rating Scales | Hamilton Rating Scale for Anxiety | Insomnia | anxiety |
| 43 | Clinician Rating Scales | Hamilton Rating Scale for Anxiety | Difficulties in concentration and memory | anxiety |
| 44 | Clinician Rating Scales | Hamilton Rating Scale for Anxiety | Depressed mood | depression |
| 45 | Clinician Rating Scales | Hamilton Rating Scale for Anxiety | General somatic symptoms muscular | anxiety |
| 46 | Clinician Rating Scales | Hamilton Rating Scale for Anxiety | General somatic symptoms sensory | anxiety |
| 47 | Clinician Rating Scales | Hamilton Rating Scale for Anxiety | Cardiovascular symptoms | anxiety |
| 48 | Clinician Rating Scales | Hamilton Rating Scale for Anxiety | Respiratory symptoms | anxiety |
| 49 | Clinician Rating Scales | Hamilton Rating Scale for Anxiety | Gastrointestinal symptoms | anxiety |
| 50 | Clinician Rating Scales | Hamilton Rating Scale for Anxiety | Genitourinary symptoms | anxiety |
| 51 | Clinician Rating Scales | Hamilton Rating Scale for Anxiety | Other autonomic symptoms | anxiety |
| 52 | Clinician Rating Scales | Hamilton Rating Scale for Anxiety | Behavior during interview | anxiety |
| 53 | Clinician Rating Scales | Hamilton Rating Scale for Anxiety | Total score | anxiety |
| 54 | Self-Report | Anxiety Sensitivity Index-3 (Baseline) | Physical concerns | anxiety |
| 55 | Self-Report | Anxiety Sensitivity Index-3 (Baseline) | Social concerns | anxiety |
| 56 | Self-Report | Anxiety Sensitivity Index-3 (Baseline) | Cognitive concerns | anxiety |
| 57 | Self-Report | Anxiety Sensitivity Index-3 (Baseline) | Total score | anxiety |
| 58 | Self-Report | Albany Panic and Phobia Questionnaire (Baseline) | Agoraphobia | anxiety |
| 59 | Self-Report | Albany Panic and Phobia Questionnaire (Baseline) | Social phobia | anxiety |
| 60 | Self-Report | Albany Panic and Phobia Questionnaire (Baseline) | Interoceptive | anxiety |
| 61 | Self-Report | Albany Panic and Phobia Questionnaire (Baseline) | Total score | anxiety |
| 62 | Self-Report | Anxiety Sensitivity Index-3 (Current) | Physical concerns | anxiety |
| 63 | Self-Report | Anxiety Sensitivity Index-3 (Current) | Social concerns | anxiety |
| 64 | Self-Report | Anxiety Sensitivity Index-3 (Current) | Cognitive concerns | anxiety |
| 65 | Self-Report | Anxiety Sensitivity Index-3 (Current) | Total score | anxiety |
| 66 | Self-Report | Albany Panic and Phobia Questionnaire (Current) | Agoraphobia | anxiety |
| 67 | Self-Report | Albany Panic and Phobia Questionnaire (Current) | Social phobia | anxiety |
| 68 | Self-Report | Albany Panic and Phobia Questionnaire (Current) | Interoceptive | anxiety |
| 69 | Self-Report | Albany Panic and Phobia Questionnaire (Current) | Total score | anxiety |
| 70 | Self-Report | Beck Depression Inventory-II (Baseline) | Total score | depression |
| 71 | Self-Report | Beck Hopelessness Scale (Baseline) | Total score | depression |
| 72 | Self-Report | Mood Disorder Questionnaire (Baseline) | Total score | (hypo)manic /bipolar |
| 73 | Self-Report | Hypomanic Symptom Checklist 32 (Baseline) | Total score | (hypo)manic /bipolar |
| 74 | Self-Report | Beck Anxiety Inventory (Baseline) | Total score | anxiety |
| 75 | Self-Report | Penn State Worry Questionnaire (Baseline) | Total score | anxiety |
| 76 | Self-Report | Liebowitz Social Anxiety Scale (Baseline) | Total score | anxiety |
| 77 | Self-Report | Obsessive-Compulsive Inventory- Revised (Baseline) | Total score | anxiety |
| 78 | Self-Report | Beck Depression Inventory-II (Current) | Total score | depression |
| 79 | Self-Report | Beck Hopelessness Scale (Current) | Total score | depression |
| 80 | Self-Report | Beck Anxiety Inventory (Current) | Total score | anxiety |

### 3-2. Network Construction Process

Diagnosis was determined based on two different strategies, that is, DSM-based primary diagnosis (DxP) and scale-based diagnosis (DxS). The DxP was assigned by psychologists within the framework of clinical psychological assessments according to the hierarchical diagnostic system of DSM-5 criteria. Regarding DxS, depression was defined as a total scale of 7 or higher on the HAMD, an anxiety disorder was diagnosed when the total scale on the HAMA was 10 or higher or the total scale on the PDSS was 8 or higher, and bipolar disorder was diagnosed when the self-report MDQ was 7 or higher or the HCL-32 scale was 12 or higher. No scale measuring psychotic symptoms was included in the data. Thereafter, SMC data were analyzed using the same methods as those described for the aforementioned UKB cohort. In the SMC cohort, the DSM-based primary diagnosis (DxP) and the scale-based diagnosis (DxS) correspond to the self-reported diagnosis (Dx) and symptom-based diagnosis (Dx) in the aforementioned UKB, respectively.

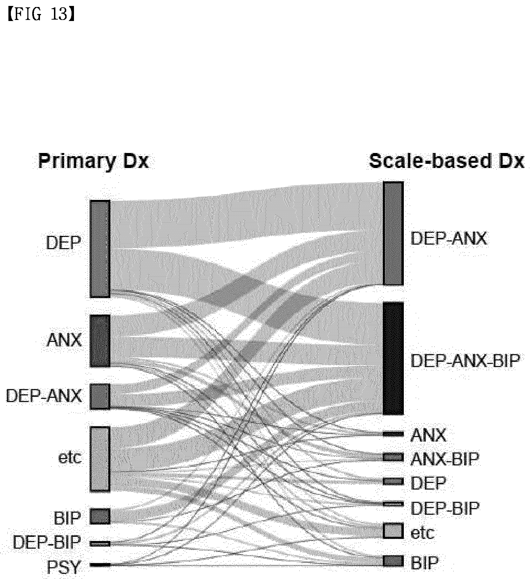

The diagnosis of patients in the SMC cohort based on DSM-5 (DxP) and scale (DxS) can be seen in Table 9 below and FIG. 13.

**[Table 9]**

| | Primary Dx | Scale-based Dx |
|---|---|---|
| DEP | 319 (39.89%) | 19 (2.26%) |
| etc | 211 (25.06%) | 44 (5.32%) |
| ANX | 167 (19.83%) | 11 (1.31%) |
| DEP-ANX | 80 (9.50%) | 338 (40.14%) |
| BIP | 47 (5.58%) | 33 (3.92%) |
| DEP-BIP | 12 (1.43%) | 11 (1.31%) |
| PSY | 6 (0.71%) | 0 (0%) |
| DEP-ANX-BIP | 0 (0%) | 365 (43.35%) |
| ANX-BIP | 0 (0%) | 21 (2.49%) |
| Total | 842 (100%) | |

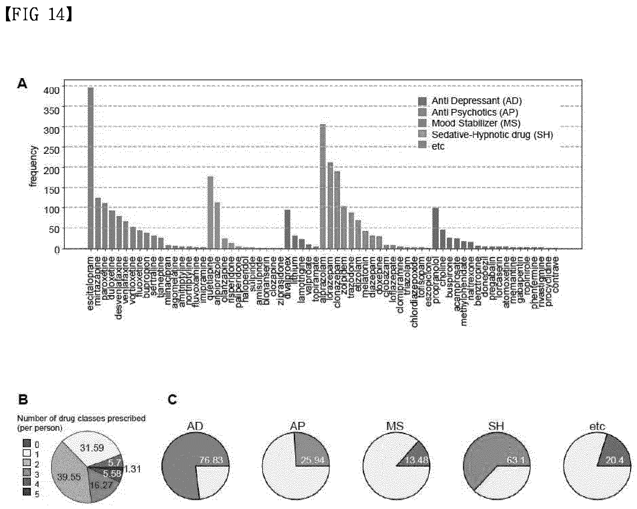

For diagnoses by DxP, patients with only depression (37.89%) were most common, followed by patients with only anxiety (19.83%). For diagnoses by DxS, patients with depression, anxiety, and bipolar disorder (43.55%) were most common, followed by patients with depression and anxiety (40.14%). As shown in FIG. 14, drug prescription data were available in 94.3% patients from the cohort. Considering the number of prescribed drug classes, 31.6%, 39.6%, and 23.2% patients were prescribed one, two, and three or more classes, respectively. The prescription probability per drug class was 76.83% for AD, 25.94% for AP, 13.48% for MS, and 63.1% for SH.

### 3-3. Clustering Analysis

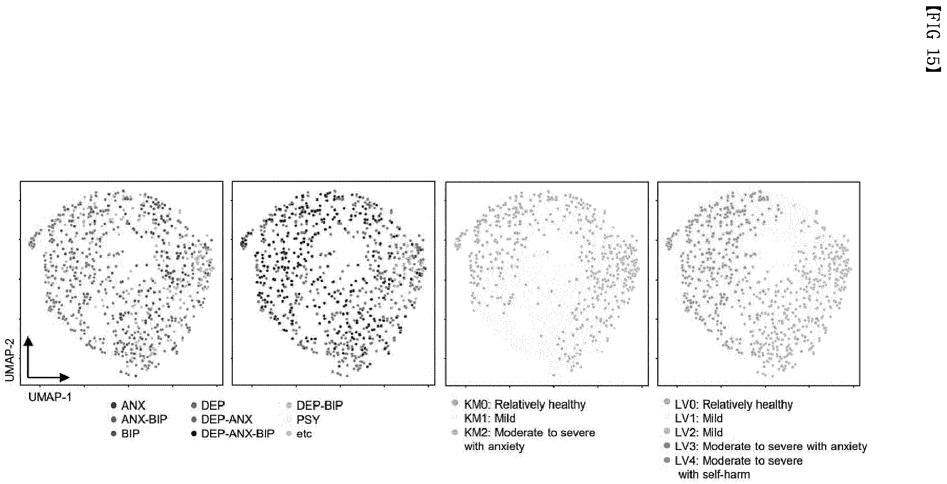

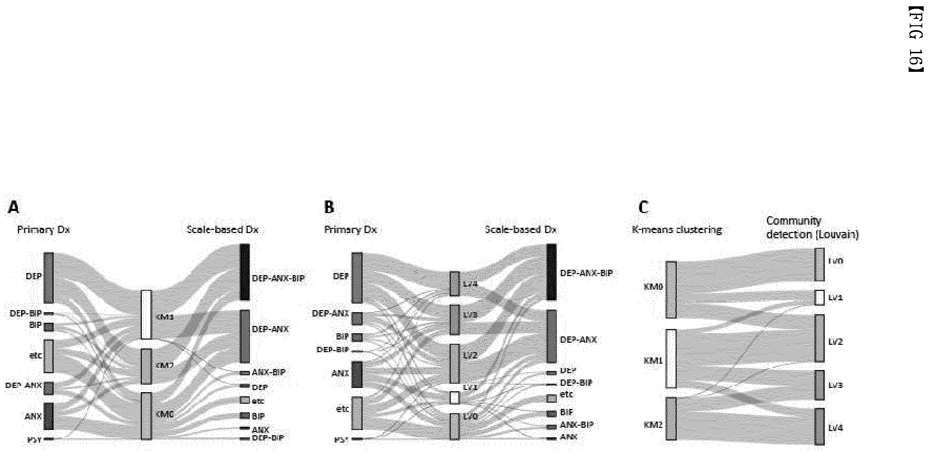

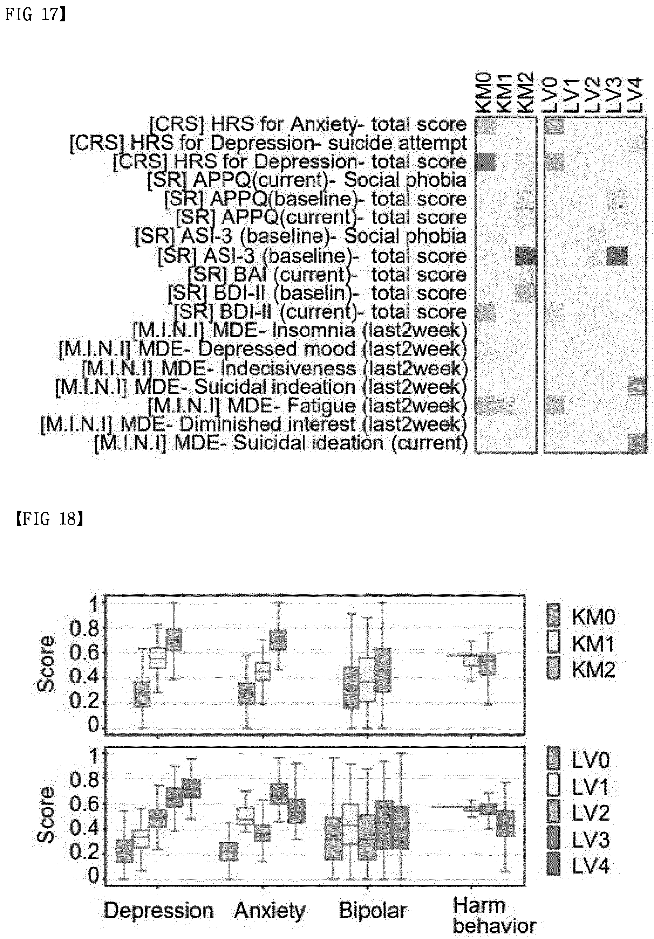

As shown in FIG. 15, three and five clusters were identified by the KM method and LV method, respectively. The results, including diagnosis mapping, feature importance pattern, and scores for each item, are presented in FIGS. 16 to 18. The clustering results reflected the severity of symptoms rather than symptom profiles; hence, the present inventors designated SMC-KM0 as "relatively healthy," SMC-KM1 as "mild," SMC-KM2 as "moderate to severe anxiety." Similarly, SMC-LV0 was designated as "relatively healthy," SMC-LV1 and SMC-LV2 as "mild," and SMC-LV3 to "moderate to severe self-harm."

### 3-4. Derivation of Drug Prescription Information

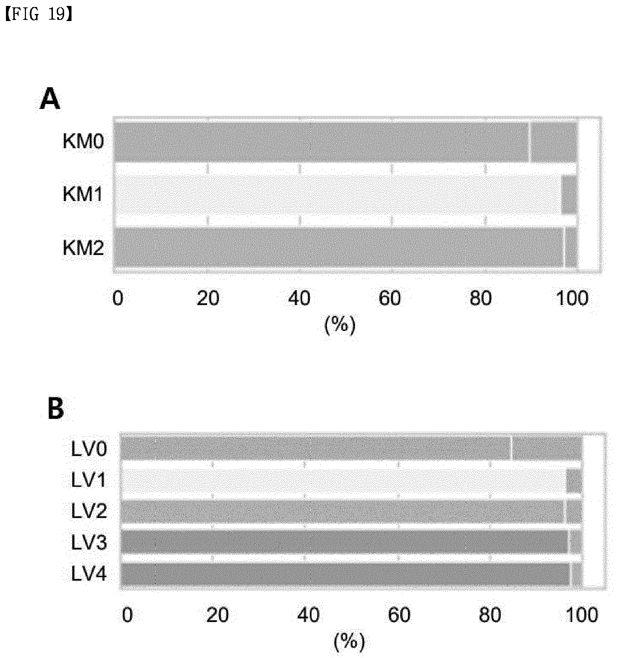

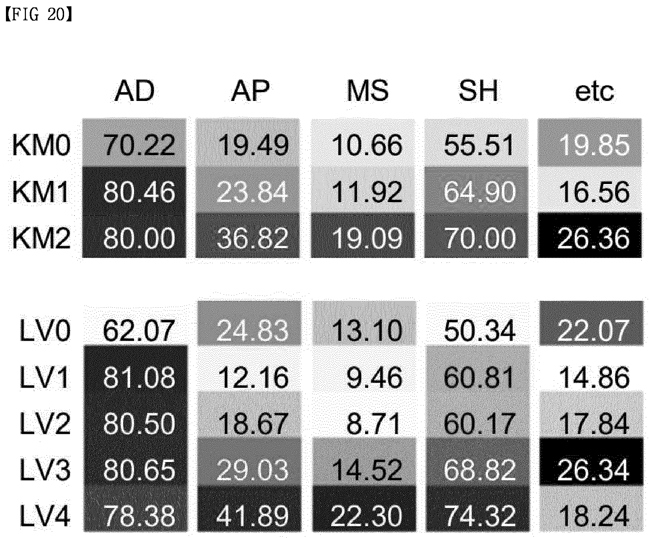

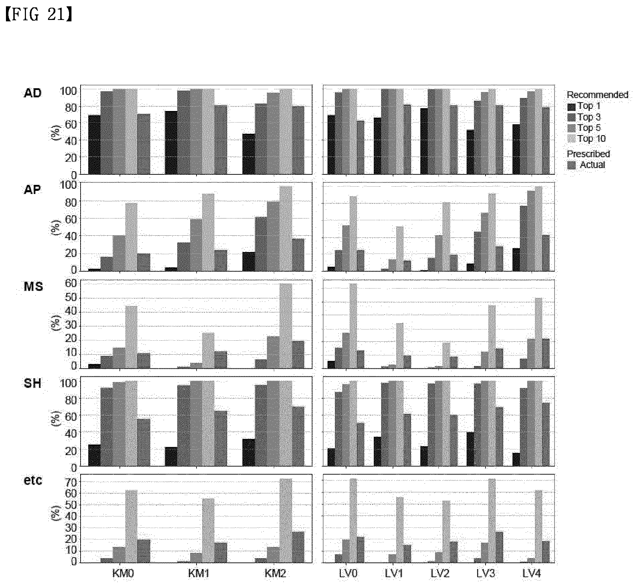

As shown in FIG. 19, it was confirmed that the overall rate of drug prescription did not differ significantly between clusters, ranging from 84.8% to 97.37%. In addition, as shown in FIG. 20, it was confirmed that, in both KM and LV clustering, AD prescription rate was lower in KM0 or LV0 than in other clusters. AP, MS, and SH generally exhibited patterns of increasing prescription rate from KM0 to KM2, and from LV1 to LV4. Also, the AP or MS prescription rate was higher in LV0 than in LV1 or LV2. This was consistent with the results from network-based recommendation, as shown in FIG. 21.

So far, the present invention has been described with reference to the preferred embodiments thereof. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A method for providing mental illness-related information and drug prescription-related information, comprising steps of:
A) constructing a system for providing mental illness-related information and drug prescription-related information,
wherein the system comprises:
a) a network information providing unit in which a network derivable from mental illness-related questionnaire information is constructed;
b) a clustering unit configured to classify mental illness groups based on mental illness-related questionnaire-based information and algorithm information based on the network information derived in a); and
c) a drug prescription information deriving unit configured to derive network-based drug prescription information based on the network information derived in a) and cluster-based drug prescription information based on mental illness group information classified in b); and
B) inputting patient's questionnaire response information into the system and providing both mental illness-related information based on the mental illness groups classified in the clustering unit b) and drug prescription-related information derived in the deriving unit c).

2. The method of claim 1, wherein the clustering unit b) classifies the mental illness groups based on k-means and Louvain algorithms.

3. The method of claim 2, wherein clustering based on the k-means algorithm is classification into 9 groups, and clustering based on the Louvain algorithm is classification into 10 groups.

4. A system for providing mental illness-related information and drug prescription-related information, comprising:
a network information providing unit in which a network derivable from mental illness-related questionnaire information is constructed;
a clustering unit configured to classify mental illness groups based on mental illness-related questionnaire-based information and algorithm information based on the network information derived in the information providing unit;
a cluster-based drug prescription information deriving unit configured to derive cluster-based drug prescription information based on mental illness group information classified in the clustering unit;
a network-based drug prescription information deriving unit configured to derive network-based drug prescription information based on network information derived in the information providing unit; and
an output unit configured to provide mental illness group information derived in the clustering unit and drug prescription information output from the network-based drug prescription information deriving unit and the cluster-based drug prescription information deriving unit.

5. The system of claim 1, wherein the clustering unit b) classifies the mental illness groups based on k-means and Louvain algorithms.

6. A computer-readable recording medium having recorded thereon a program for executing the method of claim 1 or the system of claim 4.
